(19) Europäisches Patentamt
European Patent Office
Office européen des brevets

(11) **EP 4 015 086 A1**

(12) **EUROPEAN PATENT APPLICATION**
published in accordance with Art. 153(4) EPC

(43) Date of publication:
**22.06.2022 Bulletin 2022/25**

(51) International Patent Classification (IPC):
*B02C 25/00* (2006.01)    *C07D 233/74* (2006.01)
*C07D 261/16* (2006.01)    *C07D 405/14* (2006.01)
*B02C 17/16* (2006.01)    *B02C 17/20* (2006.01)
*B02C 23/06* (2006.01)    *A61K 31/196* (2006.01)
*A61K 31/216* (2006.01)    *A61K 31/4166* (2006.01)
*A61K 31/496* (2006.01)    *A61K 31/63* (2006.01)

(21) Application number: 20854349.6

(52) Cooperative Patent Classification (CPC):
A61K 31/196; A61K 31/216; A61K 31/4166;
A61K 31/496; A61K 31/63; B02C 17/16;
B02C 17/20; B02C 23/06; B02C 25/00;
C07D 233/74; C07D 261/16; C07D 405/14

(22) Date of filing: 14.08.2020

(86) International application number:
PCT/JP2020/030862

(87) International publication number:
WO 2021/033633 (25.02.2021 Gazette 2021/08)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **16.08.2019 JP 2019149394**

(71) Applicant: Hiroshima Metal & Machinery Co., Ltd.
**Shinjuku-ku
Tokyo 160-0022 (JP)**

(72) Inventors:
• OCHII, Yuya
**Amagasaki-shi, Hyogo 660-0813 (JP)**
• TANAKA, Hironori
**Amagasaki-shi, Hyogo 660-0813 (JP)**
• IBARAKI, Tetsuharu
**Kure-shi, Hiroshima 737-0144 (JP)**
• HIRATA, Daisuke
**Kure-shi, Hiroshima 737-0144 (JP)**

(74) Representative: **Vossius & Partner
Patentanwälte Rechtsanwälte mbB
Siebertstraße 3
81675 München (DE)**

(54) **ORGANIC NANOPARTICLE PRODUCTION METHOD AND ORGANIC NANOPARTICLES**

(57) Provided is an organic nanoparticle production method comprising a step in which a mixture of beads having an average particle size at least 0.15 mm and no more than a value (mm) calculated by the formula 1.07 - 0.11 × [outer peripheral speed of the stirring rotor (m/sec)] and a slurry containing organic particles is stirred by a stirring rotor rotating at an outer peripheral speed of 7 m/sec or less in a vessel of a wet bead mill.

EP 4 015 086 A1

**Description**

Technical Field

The present disclosure relates to a method for producing organic nanoparticles using a wet bead mill. The present disclosure relates, in particular, to a method for producing nanoparticles of a poorly soluble pharmaceutical compound.

Background Art

In recent years, processes for improving functions of health foodstuff and pharmaceuticals, e.g., improvement of activity, have been attempted by grinding powders of such health foodstuff and pharmaceuticals to the order of nanometers (nano-grinding). In particular, in order to improve the activity of a poorly soluble drug, attempts for grinding drug powder to nano size is progressing. Also, refining drug particles to nano size has the effect of making the timing of drug effects constant. Thus, research on nano-sized drugs (nano-drugs) has been developing and putting into practical use.

Processes for grinding powder of organic material are generally conducted using jet mills and bead mills. Among them, grinding processes using a wet bead mill are often conducted and generally conducted as follows. A mixture (slurry) of a chemical raw material powder and a dispersion medium, in a size of several to several tens of micrometers, is prepared and charged into a bead mill containing spherical grinding media (beads). By rotating the stirring rotor at high speed in the bead mill, the mixture of the slurry and the beads is stirred, and thus, the chemical raw material powder is ground. Inorganic substances such as zirconia, alumina, hard glass, silicon carbide, and polymer materials such as polystyrene or polypropylene are used as the material of beads.

Patent Document 1 describes that the size of beads used in nano-grinding is preferably 3 mm or less, more preferably 1 mm or less. Patent Document 2 describes that powder is ground in even more smaller size by using beads of 10 to 1000 micrometers. Patent Document 3 describes that it is desirable to use beads of less than 500 micrometers in a grinding process. However, Patent Documents 1 to 3 merely describe appropriate bead size, and do not specifically describe conditions for grinding process.

Patent Document 4 describes a process using beads of 20 to 200 micrometers wherein a stirring rotor with a special shape is driven in a bead mill so that the outer peripheral speed of the stirring rotor is 3 to 8 m/sec. However, Patent Document 4 is silent about that debris from the beads and the stirring rotor gets into the slurry. In the process of Patent Document 4, the grinding efficiency may be improved by using a stirring rotor having a special shape, but the contact area between the beads and the stirring rotor is increased, and therefore, a high-speed flow is formed locally. Thus, greater amount of debris from the beads and the stirring rotor can get into the slurry.

In the field of pharmaceuticals, drugs generally have permissible content for a substance that may be harmful to health, and this also applies to nano-drugs. There is a problem in association with nano-drugs that the beads and mill components abrasion during a grinding process and contents get into the drug. In a grinding process using a wet bead mill, elements such as zirconium, yttrium, aluminum, and silicon, which are components of beads, and elements such as iron, nickel, chromium, and tungsten, which are components of metal parts of the mill, can get into the drug.

The concentration of these elements in the drug substance is required to be comply with the regulatory limits, and it is preferred to make the concentration as low as possible because the contaminants are nano-sized. Patent Document 5 describes that it is desirable that the amount of heavy metals is less than about 10 ppm in the production of pharmaceutical products, but is difficult to achieve in a grinding process using beads.

Patent Document 5 describes that beads coated with a polymer resin are used as a grinding medium in a method for reducing metal contaminants in nano-sized ground organic substance. However, even if the metal contaminants from beads can be reduced, there is a risk of contamination from polymer resins. Furthermore, metal contaminants from the bead mill device is possible, but any solution to this issue is not provided.

Prior Art Document

Patent Document

[Patent Document 1] JP H08-501073 A
[Patent Document 3] JP 2016-84294 A
[Patent Document 2] JP 2010-510988 A
[Patent Document 3] JP 2006-212489 A

[Patent Document 3] JP 2003-175341 A

Summary of Invention

Problem to be solved by the Invention

Thus, a process for nano-grinding of organic substances has been carried out based on the idea that it is sufficient if the process can be carried out efficiently, by achieving the process speed by rotating the stirring rotor at high speed. Furthermore, as described in Patent Document 5, a method to reduce metal contaminants was known but using special beads was required, and thus, the problem cannot be solved with general beads. Moreover, in the method of Patent Document 5, there was a risk of contamination from a polymer resin, which is a coating component of the beads, and heavy metals (chromium, nickel, iron, etc.), which are components of the metal parts of a bead mill device.
Therefore, for nano-grinding of powder of organic material using a wet bead mill, there was a need for a new method capable of maintaining a sufficient processing speed and significantly suppressing the concentration of contaminants from beads and the components of the bead mill device.

Means for solving the Problem

Herein disclosed are as follows:

(1) a method for preparing organic nano-particles, which comprises a step in which a mixture of beads having an average particle size at least 0.15 mm and no more than 0.9 mm and a slurry containing organic particles is stirred by a stirring rotor rotating at an outer peripheral speed of 7 m/sec or less in a vessel of a wet bead mill;

(2) a method for preparing organic nano-particles, which comprises a step in which a mixture of beads having an average particle size at least 0.15 mm and no more than a value (mm) calculated by the formula 1.07 - 0.11 × [outer peripheral speed of the stirring rotor (m/sec)] and a slurry containing organic particles is stirred by a stirring rotor rotating at an outer peripheral speed of 7 m/sec or less in a vessel of a wet bead mill;

(3) the method according to (1) or (2), wherein the beads comprise partially stabilized zirconia;

(4) the method according to any one of (1) to (3), wherein a rotating shaft for rotating the stirring rotor is inserted in the vertical direction into the vessel of the wet bead mill;

(5) a method for preparing organic nano-particles, which comprises a step in which a mixture of a slurry containing organic particles and beads is stirred by a stirring rotor in a vessel of a wet bead mill, wherein the vessel of the wet bead mill is a vertical-type cylindrical vessel with an opening at the top of the vessel, and wherein a rotating shaft for rotating the stirring rotor is inserted into the cylindrical vessel from the top of the vessel through the opening, and wherein the stirring rotor is connected with the rotating shaft;

(6) the method according to (5), wherein a slurry holder is mounted above the cylindrical vessel to connect to the vessel via a connecting pipe, and wherein a rotating shaft for rotating the stirring rotor is inserted into the vessel from the top of the slurry holder through the slurry holder and the pipe, and wherein the stirring rotor is connected with the rotating shaft, and the slurry after beads separation is discharged from the lower part of the vessel;

(7) the method according to (5) or (6), wherein the stirring rotor rotates at an outer peripheral speed of 7 m/sec or less;

(8) the method according to any one of (5) to (7), wherein the beads have an average particle size at least 0.15 mm and no more than 0.9 mm;

(9) the method according to any one of (5) to (7), wherein the beads have an average particle size at least 0.15 mm and no more than a value (mm) calculated by the formula 1.07 - 0.11 × [outer peripheral speed of the stirring rotor (m/sec)];

(10) the method according to any one of (5) to (9), wherein the beads comprises partially stabilized zirconia; and

(11) organic nanoparticles obtained by the method according to any one of (1) to (10).

Effect of the Invention

According to the method herein disclosed, contamination from beads and components of bead mill is reduced, during grinding of organic powders, such as powders of a pharmaceutical compound, into nanoparticles (for example, average particle size of 400 nanometers or less) in a wet bead mill. The method herein disclosed can prevent contamination, not only in pharmaceutical products but also in nanoparticles such as of health foodstuff and X-ray contrast agents.

Brief Description of Drawings

Fig. 1 shows a vertical-type bead mill with mechanical seal that can be used in the method herein disclosed, wherein the slurry is discharged from the lower part of the cylindrical vessel.

Fig. 2 shows a vertical-type bead mill with mechanical seal that can be used in the method herein disclosed, wherein the slurry is discharged from the upper part of the cylindrical vessel.

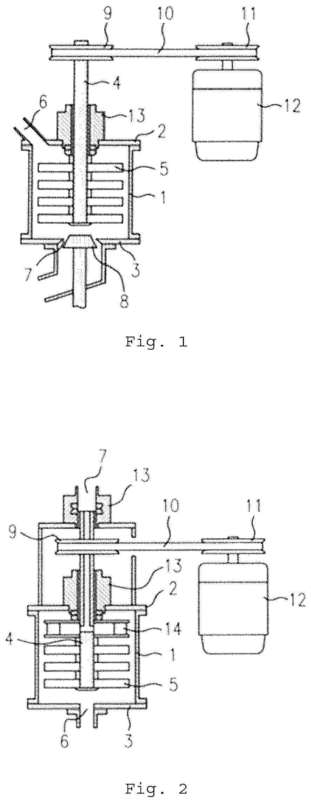

Fig. 1 shows a vertical-type bead mill without mechanical seal that can be used in the method herein disclosed, wherein the slurry is discharged from the lower part of the cylindrical vessel.

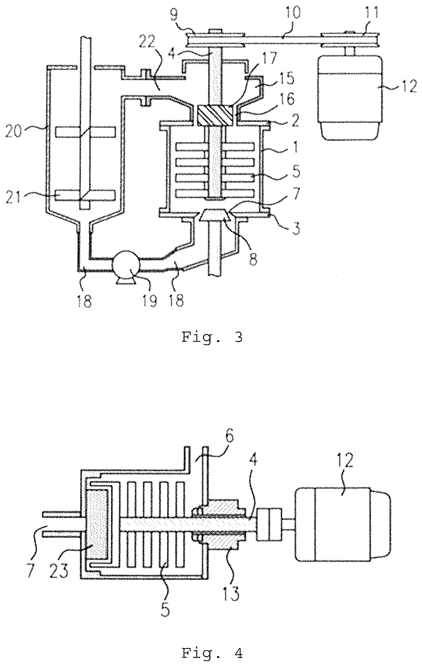

Fig. 4 shows a horizontal-type bead mill with mechanical seal that can be used in the method herein disclosed.

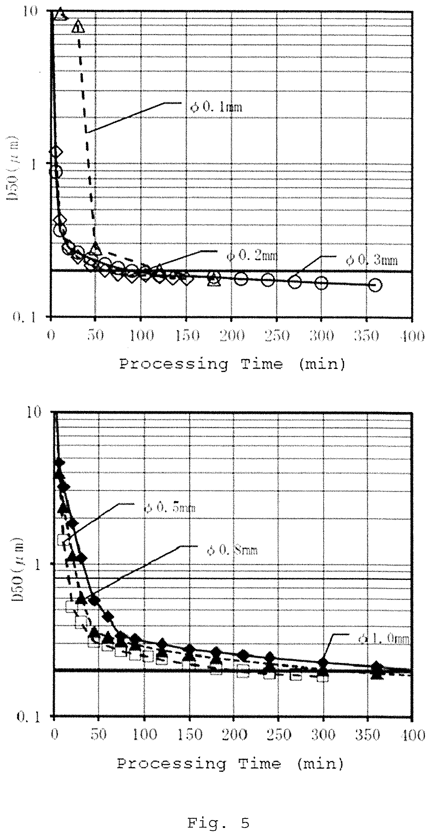

Fig. 5 shows the time course of the average particle size (D50) of the organic powder (phenytoin) during the grinding process. The upper graph shows the results using beads with a small particle size (open triangle: bead size 0.1 mm; open diamond: bead size 0.2 mm; open circle: bead size 0.3 mm), and the lower graph shows the result using beads with a large particle size (open square: bead size 0.5 mm; closed triangle: bead size 0.8 mm; closed diamond: bead size 1.0 mm) .

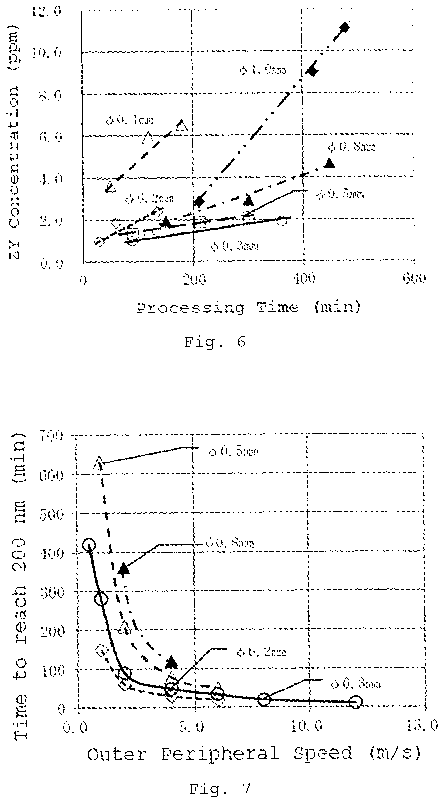

Fig. 6 shows the time course of the concentration of the contaminants (total concentration of zirconium and yttrium in the slurry: ZY concentration (ppm)) during the grinding process of the organic powder (phenytoin). Open triangle: bead size 0.1 mm; open diamond: bead size 0.2 mm; open circle: bead size 0.3 mm, open square: bead size 0.5 mm; closed triangle: bead size 0.8 mm; closed diamond: bead size 1.0 mm.

Fig. 7 shows the effect of the outer peripheral speed of the stirring rotor on the processing time. Open diamond: bead size 0.2 mm; open circle: bead size 0.3 mm; open triangle: bead size 0.5 mm; closed triangle: bead size 0.8 mm.

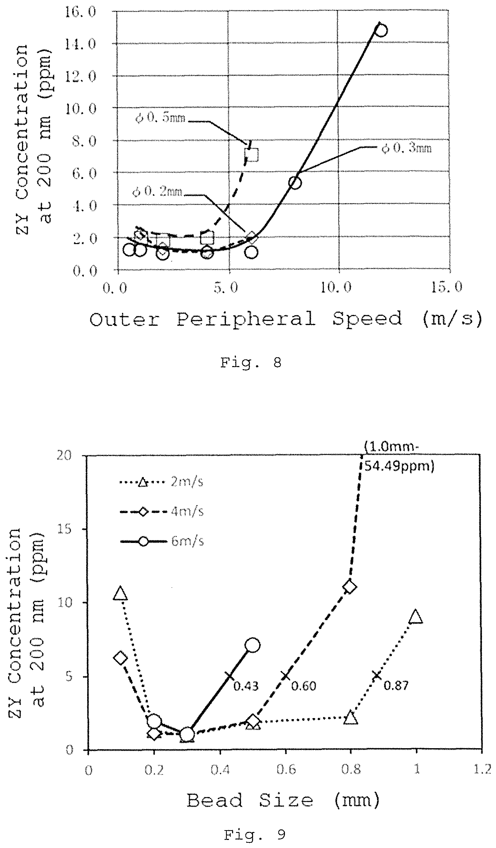

Fig. 8 shows the effect of the outer peripheral speed of the stirring rotor on the contaminant concentration. Open diamond: bead size 0.2 mm; open circle: bead size 0.3 mm; open square: bead size 0.5 mm.

Fig. 9 shows the effect of the bead size on the contaminant concentration. Open triangle: outer peripheral speed 2 m/s; open diamond: outer peripheral speed 4 m/s; open circle: outer peripheral speed 6 m/s.

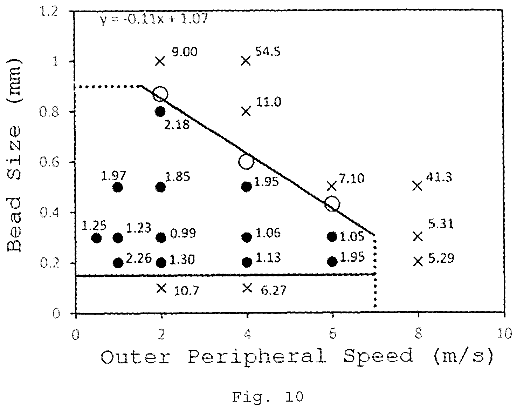

Fig. 10 shows the correlation between the bead size and the outer peripheral speed of the stirring rotor under suitable condition for grinding.

Embodiments for Carrying Out the Invention

As used herein, the "average particle size" is determined by a particle size distribution analyzer and expressed as a volume-based median diameter (D50). The average particle size herein described are obtained by measuring using HORIBA LA-950 particle size distribution analyzer. However, almost the same results are obtained when using a static laser diffraction/scattering particle size analyzer.

Unless otherwise specified, the terms "particle size" and "particle size" as used herein have the same meaning as the "average particle size".

As used herein, the term "slurry" refers to a suspension of solid organic particles of approximately 100 micrometers or less in a liquid dispersion medium. Generally, a slurry can be prepared using organic particles having an average particle size of 1 to 100 micrometers, but the method herein disclosed can be carried out even if the slurry has an average particle size of 100 micrometers or more. In the grinding process using a bead mill of the present disclosure, the processing time to achieve a particle size of 5 micrometers is very short, for example, about 3 minutes for grinding from 30 micrometers to 5 micrometers, compared to the total processing time of 45-400 minutes. Therefore, the influence of the particle size of the organic substance before the grinding on the operating conditions of the bead mill is small. In the present disclosure, the particle size of the organic particles in the slurry before the grinding process is preferably 1 to 100 micrometers, and there is no substantial influence on the operating conditions so long as it is 1 micrometer or more.

The dispersion medium used in the method herein disclosed is not particularly limited so long as it is a liquid medium in which the organic particles to be ground are essentially insoluble, and those skilled in the art can select appropriate dispersion medium, depending on the properties of the organic particles. Examples include water or various organic solvents (e.g., alcohols such as methanol, ethanol, isopropanol, butanol, ketones such as acetone, methyl ethyl ketone, methyl propyl ketone, methyl isobutyl ketone, ethers such as isopropyl ether, methyl cellosolve, glycol esters such as ethylene glycol, propylene glycol monomethyl ether acetate, ethylene glycol monoethyl ether acetate, esters such as ethyl acetate, halogenated hydrocarbons such as methylene chloride and trichloroethane, non-aromatic hydrocarbons such as cyclohexane, aromatic hydrocarbons such as toluene, and linear hydrocarbons such as normal hexane, etc.).

In the present disclosure, the "organic particle" (also referred to as "organic powder") may be any solid particles comprising an organic compound, and may be particles of any organic compound used in various fields including, but not limited to, electronic component materials, fluorescent materials, pigments, paints, pharmaceuticals, pesticides, foodstuff and the like. Examples of organic particles used in the field of pharmaceuticals include, but are not limited to, those of pharmaceutical compounds used as active ingredients of pharmaceuticals, those of additives used in pharmaceutical formulations, and those used in the production of X-ray contrasting agent.

The pharmaceutical compound is not limited, and any compound can be used. Examples include, but are not limited to, phenitoin, mefenamic acid, indomethacin, ibuprofen, itraconazole, sulfametoxazole, probucol, glyceofrubin, digoxin, perapamil, tachlorimus, dexamethasone, haloperidol, ramibdin, levamipid, alipiprazole, risperidone, ketoprofen, flurbiprofen, loxoprofen, felbinac, diferonac, acemetacin, alclofenac, fenbufen, lobenzarit, penicillamine, naproxen, pranoprofen, etodolac, cyclosporin, and the like.

The concentration of the organic particles in the slurry (also referred to as "slurry concentration") is not limited so long as the concentration provide a fluidity that allows the grinding process in a bead mill. As used herein, the slurry concentration is expressed as percent by weight of the object to be ground (organic particles) with respect to the total weight of the slurry. The slurry concentration used in the method herein disclosed may be, for example, any concentration in the range of 1 to 70% by weight, 2 to 65% by weight, 3 to 60% by weight, 4 to 55% by weight, and 5 to 50% by weight.

As used herein, the term "organic nanoparticles" refers to particles obtained by grinding the organic particles as mentioned above to a nanometer size with an average particle size of less than 1 micrometer (also herein referred to as "nano grinding"), for example, to an average particle size of 500 nanometers or less, 400 nanometers or less, 300 nanometers or less, to 200 nanometers or less, 100 nanometers or less, 50 nanometers or less, 20 nanometers or less.

According to the method herein disclosed, the mixture of the slurry and beads is stirred to be ground in a vessel of a wet bead mill by rotating a stirring rotor fixed to a rotating shaft.

The vessel of the wet bead mill that can be used by the method herein disclosed has a cross section of its inner wall is a circle that is point-symmetrical with respect to the central axis, and the diameter of the cross section of the inner wall may be constant or not, in the direction parallel to the central axis. Also, there may be a portion that is not point-symmetrical, due to a slurry supply port or the like. The wet bead mill that can be used in the method herein disclosed is that for stirring a mixture of beads and slurry in a vessel made of a material such as reinforced alumina, silicon carbide, SiAlON, partially stabilized zirconia, and stainless steel. The vessel may be equipped outside with a water jacket and water-cooled, if necessary to suppress increase of the slurry temperature inside the vessel due to the friction during the grinding process. Any capacity of the wet bead mill generally used in the art can be used in the method herein disclosed, and examples include any of 0.15L to 10L (such as 0.15L, 0.5L, 1L, 2L, 5L, 10L).

The stirring rotor may made of reinforced alumina, silicon carbide, SiAlON, hard ceramics such as partially stabilized zirconia, and a stirring rotor made of partially stabilized zirconia is preferable.

Zirconia forms a cubic crystal to become high strength, by adding calcium oxide or yttrium oxide. Furthermore, adjusting the amount to be added to zirconia slightly less than the amount that completely stabilizes the crystal, i.e., adjusting to an amount that partially stabilizes the crystal, improves the toughness and makes the ceramic material resistant to abrasion and breakage. In general, partially stabilized zirconia contains 4 to 6% by weight of yttrium oxide with respect to 94 to 96% by weight of zirconium oxide and also contains another oxide, as additives. Thus, the partially stabilized zirconia has not only high strength but also high toughness, and therefore, it is less likely to cause breakage partially. Accordingly, the stirring rotor made of partially stabilized zirconia has an advantage that cause less debris.

The method herein disclosed can be carried out using, for example, but not limited to, a wet bead mill as shown in Figs. 1 to 4, but is not limited to the apparatus, and may be carried out using a bead mill commonly used in the art.

In one embodiment, the bead mill used in the method herein disclosed is a vertical-type bead mill as shown in Fig. 1 (Apparatus 1), in which the slurry is supplied from the upper part and discharged from the lower part. The vessel of the bead mill is a vertical-type cylindrical vessel having an opening at the top, and rotary shaft 4 connected with a driving component, i.e., rotary shaft pulley 9, is inserted in the vertical direction from the top of the cylindrical vessel into the cylindrical vessel through the opening, and rotor 5 is connected with the rotating shaft 4. Mechanical seal 13 is arranged at the joint portion between the rotating shaft 4 and the cylindrical vessel. The slurry flows from the upper side to the lower side, and then, is discharged from the lower part of the cylindrical vessel after beads separation by slit-type bead separator 8.

In one embodiment, the bead mill used in the method herein disclosed is a vertical-type bead mill as shown in Fig. 2 (Apparatus 2), in which the slurry is supplied from the lower part and discharged from the upper part. The vessel of the bead mill is a vertical-type cylindrical vessel having an opening at the top, and rotary shaft 4 connected with a driving component, i.e., rotary shaft pulley 9, is inserted in the vertical direction from the top of the cylindrical vessel into the cylindrical vessel through the opening, and rotor 5 and centrifugal bead separator 14 are connected to the rotating shaft 4. Two mechanical seals 13 are mounted at the joint portions between the rotating shaft 4 and the cylindrical vessel. After bead separation by centrifugal bead separator 14, the slurry rises through the hollow flow path within the rotating shaft and is discharged from outlet 7.

As described above for Apparatus 1 and 2, in general, a wet bead mill is equipped with a mechanical seal or similar sealing parts, in order to seal the gap between the rotating shaft and the cylindrical vessel. Examples of the material of the part of the mechanical seal where the rotating portion and the fixing portion come into contact include high-strength metals and high-strength ceramics, such as iron, nickel, molybdenum, tungsten, chromium, and silicon, which may get into the slurry as the sealing part wear during the grinding process. Accordingly, the concentration of contaminants can be decreased further by caring out grinding the organic powders using a bead mill with no sealing component.

Example of the wet bead mill with no sealing component include a bead mill having a vertical-type vessel with an opening at the top, wherein a rotary shaft connected with a stirring rotor is inserted into the through the opening. A batch-type wet bead mill may be those as described above, but a circulation-type wet bead mill requires a rotating portion with a mechanism for supplying and discharging the slurry into a cylindrical vessel and with no mechanical seal. In one embodiment, an example of such apparatus is shown in Fig. 3.

The bead mill (Apparatus 3) in Fig. 3 is an example of the circulation-type wet bead mill having a vessel with similar shape and capacity to that of Apparatus 1, but with a gap at the connection of the rotating shaft and the cylindrical vessel without sealing component. Slurry holder 15 is mounted above upper lid 2, and the upper lid 2 and the slurry holder 15 are connected via connecting pipe 16. Rotary shaft 4 connected with a driving component, i.e., rotary shaft pulley 9, is inserted in the vertical direction into the cylindrical vessel through the slurry holder 15 and the connecting pipe 16. Rotor 5 is connected with the rotating shaft 4. The slurry flows from circulation tank 20 into the slurry holder 15 through slurry connecting pipe 22, and further into the cylindrical vessel through the connecting pipe 16. The slurry moves down while being ground in the cylindrical vessel and discharged from outlet 7 after bead separation by plug-type bead separator 8. The slurry is then returned to the circulation tank 20 by pump 19 through slurry pipe 18. In order to improve the slurry flow, the rotating shaft 4 may be equipped with pumping units 17 in the connecting pipe 16 so as to push the slurry downward.

A bead mill without another types of sealing component are also applicable to the method herein disclosed. Examples of such other types of bead mills include that having following structures. A cylindrical vessel has a slurry holder placed above the vessel and connected via connecting pipe. In the cylindrical vessel, a rotary shaft is inserted through the connecting pipe into the vessel at which the shaft is connected with a stirring rotor. The vessel has a slurry supply port at the bottom, and the flow of slurry raises while being ground and then separated by centrifugal bead separator at the upper part of the vessel, and further flows to the slurry holder through the hollow flow path within the rotating shaft. Similar to the apparatus of Fig. 3, a mechanism, such as pumping or rotary blades for circulating the slurry, may be equipped to allow the slurry to flow from the slurry holder to the cylindrical vessel. Such flow of the slurry can prevent beads leaking from connecting pipe. The slurry flowed downward in the connecting pipe returns to the slurry holder via the centrifugal bead separator and the hollow flow path.

The stirring rotor comprises a plurality of rod-shaped pins in the Apparatus 1 to 3, but it may be other type comprising a plurality of horizontally arranged disks in the height direction or comprising a plurality of plate-shaped objects arranged in the vertical direction.

In the method herein disclosed, the rotor speed of the stirring rotor is relatively slow, and therefore, a vertical-type bead mill is preferably used. In the case of vertical-type bead mill, centrifugal force acts in the direction perpendicular to gravity, and the force applied to the beads is almost constant in the circumferential direction inside the cylindrical vessel, and thus, there is no local excessive force and the force is applied uniformly to the beads.

The flow direction of the slurry in the cylindrical vessel of the bead mill may be either upward or downward. In cease of flowing the slurry downward, the beads can be filled downward, and opportunities for the beads to come into contact with each other are greater at the bottom of the vessel. Accordingly, it is preferable to use a vertical bead mill such as the Apparatus 1 or the Apparatus 3 in which the slurry flows from the upper side to the lower side. However, the difference is small since the flow speed of the slurry in the vertical direction is low, and the method herein disclosed can also be carried out by using a vertical-type bead mill in which the slurry flows upward.

The method herein disclosed also can be carried out using a horizontal-type bead mill. Examples of the horizontal bead mill include the bead mill (Apparatus 4) shown in Fig. 4. The Apparatus 4 has rotation shaft 4 in the horizontal direction wherein a plurality of stirring rotors 5 with petal-shaped and perforated are joined to the shaft in parallel with the rotation direction so that the stirring rotor 5 stirs the slurry and beads. The slurry as processed is discharged out of the cylindrical vessel after the beads separation by screen 23.

In the case of a horizontal-type bead mill, the directions of centrifugal force and gravity depend on the position in the circumferential direction in the cylindrical vessel. At the top of the side of the cylindrical vessel, the centrifugal force is subtracted by gravity, reducing the force pressing the beads. On the other hand, at the bottom, gravity is applied to the centrifugal force, increasing the force pressing the beads. The method herein disclosed is carried out under a condition that the outer peripheral speed of the stirring rotor (also herein referred to as "outer peripheral speed") is relatively low, and thus, the centrifugal force is small. Accordingly, the phenomenon as mentioned occurs greatly, and the beads are difficult to rise to the top of the cylindrical vessel, slowing the processing speed. For this reason, a horizontal-type bead mill, particularly in case that the outer peripheral speed is low, would result slightly larger amount of contaminants than that in case of using a vertical-type bead mill, but it can be used in the method herein disclosed.

In a circulation-type wet bead mill, the slurry is typically processed over 3 to 10 minutes per cycle and the circulation processing is carried out approximately 5 to 50 times. The processing time is typical 30 to 400 minutes, but may be shorter or longer depending on the capacity of the mill.

The beads used in the method herein disclosed are not limited so long as that commonly used for grinding process using a wet bead mill, and a person skilled in the art can select appropriately, taking into account various factors, such as spec of the bead mill, characteristics of the material to be ground (for example, hardness, density and size of the particles), target particle size after processing, viscosity of the slurry, and the like.

Examples of the material of the beads used in a bead mill include, but are not limited to, glass, alumina, zircon (zirconia-silica ceramics), zirconia, and steel. Zirconia is preferable as a material for beads because of its high hardness and occurring less debris due to beads deterioration. In particular, beads made of partially stabilized zirconia are particularly preferable because, as described above, not only its high strength but also high toughness, and therefore, it is less likely to cause breakage partially.

In one embodiment, beads made of partially stabilized zirconia are used in the methods of the present disclosure. The beads made of partially stabilized zirconia are also herein simply referred to as "beads".

As used herein, the term "bead filling factor" refers to the percent of the apparent volume (% by volume) of the beads with respect to the effective volume of the cylindrical vessel (i.e., the internal volume of the cylindrical vessel minus the volume of the stirring rotor) of the bead mill.

A person skilled in the art can appropriately select the bead filling ratio, taking into consideration of various factors such as the spec and operating conditions of the bead mill and the viscosity of the slurry. Typically, the bead filling ratio may be set within the range of 10 to 95% by volume, for example, 15 to 95% by volume, 25 to 90% by volume, 35 to 90% by volume, 50 to 90% by volume, 75 to 90% by volume.

A person skilled in the art can appropriately determine the amount of slurry charged into the bead mill, according to the spec of the bead mill (for example, the capacity of the grinding chamber of the bead mill to be used), operating conditions, and the like.

In general, organic powder is relatively soft and can be ground even though the collision energy of beads is small, and therefore, the average particle size of beads (also herein referred to as "bead diameter") may be relatively small in the method herein disclosed. Further, the smaller the particle size of the beads, the larger the specific surface area and the faster the grinding speed. There are two factors casing increased bead wear, one is due to the specific surface area of the beads and the other is due to the mass of the single bead. In the former, the larger the particle size of the beads, the less wear. In the latter, the smaller the particle size of the beads, the less wear. Taking into account both, moderate size of the beads is considered as occurring less wear. In the method herein disclosed, beads having an average particle size at least 0.15 mm and no more than 0.9 mm, such as commercially available as particle size standards of 0.15 mm, 0.2 mm, 0.3 mm, 0.4 mm, 0.5 mm, 0.6 mm, 0.7 mm, 0.8 mm, or 0.9 mm, may be used.

Generally, beads have the surface area of much larger than that of the bead mill components. For example, in a bead mill with the effective internal volume of 200 ml, the total area of the inner surface of the cylindrical vessel and the stirring rotor is about $10^5$ mm$^2$, while the total surface area of the beads is on the order of $10^6$ to $10^7$ mm$^2$, and therefore, the wear due to contact between the beads is much larger than that of the bead mill components.

In the method herein disclosed reducing the wear of beads, the lowest contaminant's concentration that can be achieved will depend on the shape of the stirring rotor. However, in principle, the method herein disclosed can be applied to stirring rotors of all the shape, and the effect of the method is expected even with a stirring rotor having a complicated shape.

Examples of the stirring rotor of the wet bead mill used in the method herein disclosed include a stirring rotor composed of rod-shaped pins at a point-symmetrical position to the rotation direction, a stirring rotor composed of a plurality of plates in parallel with the rotation direction, a stirring rotor composed of a plurality of plates in parallel with the rotation axis, and the like. In a stirring rotor composed of stirring pins, the stirring pins are not always a columnar shape and may have a plate shape, which is not needed to be a simple plate shape.

As used herein, the "outer peripheral speed" refers to the outer peripheral speed of the stirring rotor during rotation.

Under the same condition in the beads size, the faster the outer peripheral speed of the stirring rotor, the shorter the processing time. In the method herein disclosed, preferred outer peripheral speed is 1 m/sec or more, but it is possible to grind organic particles to a particle size of about 200 nanometers even if the outer peripheral speed is 0.5 m/sec.

The outer peripheral speed of the stirring rotor also affects the wear of the beads and the stirring rotor. In the method herein disclosed, when the outer peripheral speed of the stirring rotor is 7 m/sec or less, the concentration of contaminants from beads and the stirring rotor can be significantly suppressed with maintaining a sufficient processing speed. The outer peripheral speed of the stirring rotor in the method herein disclosed is may be in the range of 0.5 m/sec to 7 m/sec (for example, 0.5 m/sec, 1 m/sec, 2 m/sec, 3 m/sec, 4 m/sec, 5 m/sec, 6 m/sec, 7 m/sec).

In one embodiment, the method herein disclosed comprising a step of grinding with partially stabilized zirconia beads having an average particle size at least 0.15 mm and no more than a value (mm) calculated by the formula 1.07-0.11 × [outer peripheral speed of the stirring rotor (m/sec)] (mm), thereby the concentration of contaminants from the beads and the stirring rotor can be significantly suppressed with maintaining a sufficient grinding rate.

In one embodiment, the amount of contaminants contained in the organic nanoparticles obtained by the method herein disclosed is, for example, 0.0001 ppm or more and less than 50 ppm, 0.0001 ppm or more and less than 40 ppm, 0.0001 ppm or more and less than 30 ppm, 0.0001 ppm or more and less than 20 ppm, and 0.0001 ppm or more and less than 10 ppm, with respect to the total weight of the obtained particles. In the present disclosure, the concentration of the contaminant is expressed as ppm (mass ppm) of the amount of the contaminant, with respect to the mass of the entire slurry. In the present disclosure, "ZY concentration" is expressed as ppm (mass ppm) of the total mass of zirconium and yttrium with respect to the mass of the entire slurry. In one embodiment, the ZY concentration in the slurry after the grinding process by the method herein disclosed is about 5 ppm or less.

The concentration of contaminants in the slurry can be determined by any method commonly used in the art, such as inductively coupled plasma mass spectrometry (ICP-MS).

In the field of pharmaceuticals, as described in Patent Document 5, there is a guideline to reduce the concentration of heavy metals in the drug substance to 10 ppm or less. Therefore, for example, if the concentration of the slurry to be subjected to the grinding process is 50% by weight, then desired concentration of heavy metals in the slurry after the grinding process is about 5 ppm or less. However, the present disclosure provides a method for grinding organic powder with a wet bead mill rapidly with reducing the concentration of contaminants, and it is not always required to meet the guideline.

In carrying out the method herein disclosed, additives can be added to the slurry as needed. For example, a dispersant can be added to the slurry for the purpose of improving the dispersibility of the organic particles in the slurry, preventing aggregation, or stabilizing the dispersion.

The dispersant can be appropriately selected taking into consideration of various factors such as the properties of the organic particles and the dispersion medium, the spec of the bead mill and the operating conditions. Examples of the dispersant include surfactants such as carboxylate (e.g., fatty acid salt, etc.), sulfonate (e.g., sodium linear alkylbenzene sulfonate, etc.), phosphate (e.g., monoalkyl phosphate, etc.), sulfate ester salt (e.g., sodium lauryl sulfate, etc.) and polymer compounds such as hydroxypropyl cellulose (HPC), hypromellose (hydroxypropyl methyl cellulose (HPMC)), methyl cellulose (MC) and polyvinylpyrrolidone (PVP). The amount of the dispersant can be appropriately determined by a person skilled in the art according to conventional procedures.

Other conditions of the grinding process to be carried out in the method herein disclosed can be set appropriately by a person skilled in the art, taking into consideration of various factors (e.g., properties of the organic material, type of the dispersion medium, viscosity of the slurry, particle size of the nanoparticles obtained after grinding, and the grinding efficiency, etc.).

The slurry discharged from the bead mill may be dried to remove the dispersion medium, according to a conventional procedure in the art, to afford powders comprising organic nanoparticles.

In a further aspect of the present disclosure, organic nanoparticles obtained by the methods of the present disclosure are provided.

In one embodiment, the organic nanoparticles of the present disclosure comprise a pharmaceutical compound.

The form of the organic nanoparticles obtained by the method herein disclosed is not limited, and may be in a slurry obtained by the method herein disclosed or may be powders obtained after dryness of the slurry.

In a further aspect of the present disclosure, a composition or material comprising the organic nanoparticles obtained by the methods of the present disclosure is provided. Examples of such compositions or materials include materials for electronic device including dielectrics, piezoelectrics, and magnetic materials, phosphors, materials for battery electrode, fluorescent materials, paints, raw materials for fine ceramics, abrasives, pharmaceuticals, pesticides, and foodstuffs.

In a further aspect of the present disclosure, a pharmaceutical composition comprising the organic nanoparticles obtained by the methods of the present disclosure is provided.

The pharmaceutical composition of the present disclosure can be prepared using the organic nanoparticles obtained by the method herein disclosed by several steps as appropriately arranged according to desired dosage commonly used in the field of pharmaceutical formulation (e.g., granulation, sizing, tableting, coating, etc.).

As one embodiment, the operation method of the bead mill used in the method herein disclosed is described using the Apparatus 1. The slurry is supplied from the slurry supply port 6 into the vessel composed of cylinder 1, upper lid 2 and lower lid 3. The mixture of slurry and beads is stirred by the stirring rotor 5 connected with rotating shaft 4. The stirring rotor 5 is composed of a plurality of rod-shaped pins. The slurry flows down in the cylindrical vessel from the slurry supply port 6 at a speed generally 10 to several tens of mm/sec. The organic particles in the slurry are ground by stirring rotor 5. The processed slurry is discharged from slurry outlet 7 to the outside of the cylindrical vessel, after beads separation by plug-type bead separator 8. In order to secure the pressure within the bead mill, mechanical seal 13 is mounted around the rotating shaft 4. Although not shown in Fig. 1, the discharged slurry is pumped to flow through the pipe and returns to the circulation tank. As described above, the slurry is typically processed while circulating between the circulation tank and the bead mill.

The following Test Examples and Examples are intended to explain this disclosure in more detail and should not be construed as limiting its scope in any way.

Examples

Test Example 1: Effect of bead diameter on processing time

The grinding process was carried out by stirring a slurry (500 g) containing phenitoin (5 wt%, raw material particle size: 16 to 20 pm, Shizuoka Caffein Co., Ltd.) and dispersants (polyvinylpyrrolidone (3 wt%) and sodium lauryl sulfate (0.25 wt%)), with partially stabilized zirconia beads (YTZ ball manufactured by Nikkato Corporation, which was the same as used hereinafter) having different average particle seize (particle size standard), at outer peripheral speed of 2 m/sec of the stirring rotor in a wet bead mill (Apex Mill 015, manufactured by Hiroshima Metal & Machinery, which is Apparatus 1 as described above and referred to as "Apparatus 1" in the following Examples and Test Examples). Sampling was carried out at a predetermined time point during the grinding process, and the particle size of the phenytoin particles and the concentration of contaminants (the total concentration of zirconium and yttrium; herein referred to as "ZY concentration") in the samples were measured.

The particle size the phenytoin particles was determined using LA-950 (HORIBA, Ltd.).

Measurement conditions:

Particle refractive index: 1.610 (phenytoin)
Set Zero: 60 seconds
Measurement time: 60 seconds
Number of measurements: 2 times
Shape: non-spherical
Solvent refractive index: 1.333 (water)
Ultrasound: None
Particle size standard: by volume

The concentration of contaminants in the slurry was measured according to the following procedure (the same applies to the following Examples and Test Examples).

The sample after the grinding process (0.5 g) was weighed in a metal-free container and added with internal standard substance (Co) and a mixed solution of NMP/HCl/HNO$_3$ (90:5:5), and dissolved by ultrasonic irradiation. The sample solution was subjected to inductively coupled plasma mass spectrometry (ICP-MS) (iCAPQ™, Thermo Fisher Scientific K.K.) to determine the concentration (ppm by weight) of contaminants (zirconium and yttrium) in the sample.

Measurement conditions:

Elements to be measured: Zr (m/z = 90), Y (m/z = 89) Nebulizer: Coaxial nebulizer
Spray chamber: Cyclone type
Spray chamber temperature: constant around 3°C
Injector inner diameter: 1.0 mm
Sample introduction: Natural suction
High frequency power: 1550 W
Cooling gas flow rate: 14 L/min
Auxiliary gas flow rate: 0.8 L/min
Measurement mode: KED
Collision gas: helium
Additive gas: oxygen
Perista pump rotation speed: 20 rpm
Integration time: 0.1 seconds
Accumulation number: 3 times

The results are shown in Fig.5 and Fig.6. The ZY concentration is expressed by ppm of the mass with respect to the weight of the slurry after grinding process (the same is applied hereinafter).

As shown in Fig.5, when the bead diameter is 0.2 mm or more, the grinding process proceeds rapidly until the particle size of phenytoin reaches about 400 nanometers. However, the speed to reach 400 nanometers or less decreases, and the effect of the bead diameter on the processing speed increases. The smaller the bead diameter, the faster the processing speed, and the time required to reach 200 nanometers is the shortest when the bead diameter is 0.2 mm (open diamond in Fig. 5) and 0.3 mm (open circle in Fig. 5). When the bead diameter is 0.1 mm (open triangle in Fig. 5), the initial processing speed was slow, and a very small amount of particles of 1 micrometer or more remained, but it was possible to grind up to 200 nanometers. Thus, organic powder is available to be ground even small-diameter beads having a small collision energy, since the organic powder is relatively soft as described above. In addition, small-diameter beads have a large specific surface area, so that the processing speed is high. When the bead diameter is 0.1 mm, the impact force of the beads is small, and therefore, it takes time to grind powder of several tens of micrometers or more, but the grinding rapidly proceeded once the particle size reached 8 micrometers or less.

As shown in Fig. 6 plotting ZY concentration versus processing time, good results were obtained when the bead diameter was 0.2 to 0.8 mm, and 0.3 mm gave the best result (open circle in Fig. 6). When using beads having a bead diameter of 0.1 mm (open triangle in Fig. 6) and 1 mm (closed diamond in Fig. 6), the concentration of contaminants (ZY concentration) was high due to the wear of the beads. As described above, there are two factors causing the wear of beads, one causes increased wear due to the specific surface area of the beads and the other causes increased wear due to the mass of the beads. The beads having a bead diameter of 0.1 mm suffered increased wear because it was greatly affected by the specific surface area. The beads having a bead diameter of 1 mm suffered increased wear because it was greatly affected by the mass of each bead and the collision energy between the beads was large.

Test Example 2: Effect of outer peripheral speed on processing time

The correlation between the outer peripheral speed and the processing time in the grinding process of the present disclosure was investigated. In the Apparatus 1, using partially stabilized zirconia beads (bead filling ratio: 75%) having different bead diameters of 0.2 mm to 0.8 mm, a slurry (500 g) containing 5 wt% of phenitoin (raw material particle size: 16 to 20 $\mu$m) and dispersants (polyvinylpyrrolidone (3 wt%) and sodium lauryl sulfate (0.25 wt%)) was ground to reach an average particle size of around 200 nanometers. The results are shown in Fig.7.

Under the same bead diameter, the faster the outer peripheral speed, the shorter the processing time. When beads having a diameter of 0.3 mm were used (open circle in Fig. 7), it was possible to ground up to 200 nanometers in about 420 minutes even at an outer peripheral speed of 0.5 m/sec.

Test Example 3: Effect of outer peripheral speed on contaminant concentration

In the Apparatus 1, a slurry (500 g) containing 5 wt% of phenitoin (raw material particle size: 16 to 20 $\mu$m) and dispersants (polyvinylpyrrolidone (3 wt%) and sodium lauryl sulfate (0.25 wt%)) was ground to reach an average particle size of around 200 nanometers using partially stabilized zirconia beads (bead filling ratio: 75%) as described in Test Example 2, at different outer peripheral speed. The concentration of contaminants (ZY concentration) when the average particle size of the phenytoin particles reached 200 nanometers was measured. The results are shown in Fig.8.

Test Example 4: Effect of bead diameter on contaminant concentration

In the Apparatus 1, a slurry (500 g) containing 5 wt% of phenitoin (raw material particle size: 16 to 20 $\mu$m) and dispersants (polyvinylpyrrolidone (3 wt%) and sodium lauryl sulfate (0.25 wt%)) was ground to reach an average particle size of around 200 nanometers as described in Test Example 2, using partially stabilized zirconia beads having different bead diameter (bead filling ratio: 75%). The grinding processes were carried out at an outer peripheral speed of the stirring rotor 2 m/sec, 4 m/sec, and 6 m/sec, respectively. The concentration of contaminants (ZY concentration) when the average particle size of the phenytoin particles reached 200 nanometers was measured. The results are shown in Fig.9.

The bead diameters used were 0.1 to 1 mm. The ZY concentration was the lowest when the bead diameter was 0.2 to 0.3 mm at every outer peripheral speed, and the ZY concentration was high when the bead diameter was small and when the bead diameter was large. As shown in Fig. 9, the ZY concentration increased greatly between 0.8 mm and 1.0 mm of the bead diameter at 2 m/sec of the outer peripheral speed (open triangle in Fig. 9), between 0.5 mm and 0.8 mm of the bead diameter at 4 m/sec of the outer peripheral speed (open diamond in Fig. 9) and between 0.3 mm and 0.5 mm of the bead diameter at 6 m/sec of the outer peripheral speed (open circle in Fig. 9). As shown in Fig. 9, for each peripheral speed, the value of the bead diameter at which the ZY concentration reaches 5 ppm was determined from the intersection of the straight line connecting the data plots of the bead diameters and the horizontal straight line showing the ZY concentration of 5 ppm, and the obtained values were put in the graph. The ZY concentration was also increased when the bead diameter was small, as found that the ZY concentration when the bead diameter was 0.1 mm was higher than that when the bead diameter was 0.2 mm. Judging from the graph of Fig. 9, the lower threshold of the bead diameter at which the ZY concentration exceeds 5 ppm is approximately 0.15 mm. Thus, the upper threshold of the bead diameter at which the ZY concentration greatly increases is a value that varies depending on the outer peripheral speed, while the lower threshold is approximately 0.15 mm.

Test Example 5: Correlation between bead diameter and outer peripheral speed

In the Apparatus 1, a slurry (500 g) containing 5 wt% of phenitoin (raw material particle size: 16 to 20 $\mu$m) and dispersants (polyvinylpyrrolidone (3 wt%) and sodium lauryl sulfate (0.25 wt%)) was ground to reach an average particle size of around 200 nanometers, using partially stabilized zirconia beads having different bead diameter as shown in Table 1 (bead filling ratio: 75%) at different outer peripheral speed as shown in Table 1. The concentration of contaminants (ZY concentration) when the average particle size of the phenytoin particles reached 200 nanometers was measured.

Table 1

| outer peripheral speed (m/s) | bead diameter (mm) | ZY concentration (ppm) |
|---|---|---|
| 0.5 | 0.3 | 1.25 |
| 1.0 | 0.2 | 2.26 |
| 1.0 | 0.3 | 1.23 |
| 1.0 | 0.5 | 1.97 |
| 2.0 | 0.1 | 10.7 |
| 2.0 | 0.2 | 1.30 |
| 2.0 | 0.3 | 0.99 |
| 2.0 | 0.5 | 1.85 |
| 2.0 | 0.8 | 2.18 |
| 2.0 | 1.0 | 9.00 |
| 4.0 | 0.1 | 6.27 |
| 4.0 | 0.2 | 1.13 |
| 4.0 | 0.3 | 1.06 |
| 4.0 | 0.5 | 1.95 |
| 4.0 | 0.8 | 11.0 |
| 4.0 | 1.0 | 54.5 |
| 6.0 | 0.2 | 1.95 |
| 6.0 | 0.3 | 1.05 |
| 6.0 | 0.5 | 7.10 |
| 8.0 | 0.2 | 5.29 |
| 8.0 | 0.3 | 5.31 |
| 8.0 | 0.5 | 41.3 |

As shown in Fig. 10, each data in the above table are plotted with the horizontal axis as the outer peripheral speed and the vertical axis as the bead diameter, and the values of the ZY concentration (unit: ppm) are added to each data point. The upper threshold of the bead diameter at which the ZY concentration in the slurry reaches 5 ppm as obtained above from Fig. 9 for each outer peripheral speed (0.87 mm at 2 m/s, 0.60 mm at 4 m/s, and 0.43 mm at 6 m/s) are shown as open circles in Fig. 10. As shown in Fig. 10, there is a linear relationship between the upper threshold of the bead diameter at which the ZY concentration in the slurry reaches 5 ppm (Do) and the outer peripheral speed of the stirring rotor, which is represented by the following formula:

```
Do (mm) = 1.07 - 0.11 × [outer peripheral speed of stirring
rotor (m/sec)]
```

Further, the lower threshold of the bead diameter at which the ZY concentration in the slurry reaches 5 ppm is approximately 0.15 mm as shown in Test Example 4, and is shown by the horizontal straight line in Fig. 10.

As shown in Fig. 10, the ZY concentration in the slurry is suppressed to 5 ppm or less under the condition corresponding to the data points inside the area between the two straight lines. On the other hand, under the condition corresponding to the data points outside the area between the two straight lines, the ZY concentration increased considerably.

Test Example 6: Effect of bead filling ratio

Using partially stabilized zirconia beads having bead diameter of 0.3 mm at different bead filling ratio, a slurry (500 g) containing 5 wt% of phenitoin (raw material particle size: 16 to 20 $\mu$m) and dispersants (polyvinylpyrrolidone (3 wt%) and sodium lauryl sulfate (0.25 wt%)) was ground as described in Test Example 1, at outer peripheral speed of the stirring rotor of 2 m/s until the average particle size reaches around 200 nanometers. The concentration of contaminants (ZY concentration) when the average particle size of the phenytoin particles reached 200 nanometers was measured.

The processing time and ZY concentration were 600 minutes and 0.50 ppm at the filling ratio of 25%, 330 minutes and 0.70 ppm at the filling ratio of 35%, 90 minutes and 0.99 ppm at the filling ratio of 75%, and 90 minutes and 1.4 ppm at the filling ratio of 90%, respectively. At the filling ratio of 25%, the ZY concentration was low while the processing time was long. At the filling ratio of 90%, there is no difference in the processing time from the filling ratio of 75%, while the ZY concentration increased slightly.

Test Example 7: Effect of slurry concentration

In the Apparatus 1, a slurry containing different concentrations of phenytoin (raw material particle size: 16 to 20 $\mu$m) was ground to reach an average particle size of around 200 nanometers as described in Test Example 1 using partially stabilized zirconia beads having diameter of 0.3 mm (bead filling ratio: 75%) at an outer peripheral speed of 2 m/sec. The processing time to reach 200 nanometers and the concentration of contaminants (ZY concentration) when reached 200 nanometers are shown in the following table.

Table 2

| Operating condition<br>Bead diameter: 0.3 mm<br>Outer peripheral speed: 2 m/s<br>Bead filling ratio: 75% | | |
| --- | --- | --- |
| Slurry Concentration mass% | Time to reach 200 nm | Contaminant Concentration ppm |
| 5 | 90 | 0.99 |
| 30 | 90 | 1.09 |
| 40 | 90 | 1.17 |

There was no significant difference in the time for grinding up to 200 nanometers even though the phenytoin concentration in the slurry was changed. Also, there was no significant difference in the ZY concentration. Thus, the slurry concentration did not affect the ZY concentration. At a high concentration of 50 wt%, the fluidity of the slurry deteriorated, but it was enabled to carry out the grinding process.

Examples 1 to 31

Slurries of different organic particles (phenytoin, sulfamethoxazole, fenofibrate, mefenamic acid, itraconazole) were ground using the bead mills shown in Figs. 1 to 4 (Apparatuses 1 to 4). The bead mills used are as follows (the zirconia recited below contain yttrium as an additive).

Apparatus 1: Apex Mill 015 (manufactured by Hiroshima Metal & Machinery). The materials of the parts in contact with the drugs are tungsten carbide, nickel (mechanical seal), zirconia-containing reinforced alumina (stator), zirconia (rotor) and perflo (O-ring).

Apparatus 2: Ultra Apex Mill 015 (manufactured by Hiroshima Metal & Machinery). The materials of the parts in contact with the drugs are tungsten carbide, nickel (upper and lower mechanical seals), zirconia-containing reinforced alumina (stator), zirconia (separator, rotor) and perflo (O-ring).

Apparatus 3: Experimental prototype. The materials of the parts in contact with the drugs are zirconia-containing reinforced alumina (stator), zirconia (rotor), SUS316L (pumping unit) and perflo (O-ring).

Apparatus 4: Dyno Mill Research Lab [small wet disperser/grinder compatible for fine beads] (manufactured by Shinmaru Enterprises Corporation). The materials of the parts in contact with the drugs are zirconia (containing hafnium) (accelerator, wear bush), SiC (silicon carbide) (grinding cylinder), nickel, hard chromium plating (screen), Viton (registered trademark) (O-ring).

Table 3 shows the conditions and results of the grinding process of each Example. Further, Comparative Example 1 using reinforced alumina beads and Comparative Examples 2 to 6 in which the outer peripheral speed or the bead diameter is outside the range of the conditions of the present disclosure are shown as comparative examples. The concentrations of contaminants are shown by mass ppm with respect to the weight of the slurry after grinding process, respectively for zirconium and yttrium which are the bead components, reinforced alumina (aluminum) which is the material of the vessel of the bead mill, and iron, nickel, chromium and tungsten which are the main materials of the metal parts used in the bead mill.

Table 3

| Ex. No. | Appratus Spec | | | | | Operating Condition | | | | | | | | | Contaminant Concentration | | | | | | | | |
|---|---|---|---|---|---|---|---|---|---|---|---|---|---|---|---|---|---|---|---|---|---|---|---|
| | Apparatus No. | Rotation Axis | Stator Material | Rotor Shape | Sealling | Bead Material | Bead Size | Outer Speed | Bead Filling Ratio | Organic particle | Slurry Conc. | Starting Particle Size | Final Particle Size | Process Time | Zr | Y | Zr Y Total | Al | Fe | Ni | Cr | W | Total |
| | | | | | | | mm | m/s | % | | mass% | μm | nm | min | ppm | ppm | ppm | ppm | ppm | ppm | ppm | ppm | ppm |
| Ex.1 | 1 | vertical | AL | Pin | Yes | ZY | 0.2 | 2 | 75 | phenitoin | 5 | 16-20 | 246 | 30 | 0.71 | 0.26 | 0.97 | 0.49 | 0.09 | 1.73 | 0.00 | 0.00 | 3.28 |
| Ex.2 | 1 | vertical | AL | Plate | Yes | ZY | 0.2 | 4 | 50 | phenitoin | 5 | 16-20 | 201 | 60 | 0.89 | 0.19 | 1.08 | 0.37 | 0.04 | 2.71 | 0.00 | 0.03 | 4.23 |
| Ex.3 | 1 | vertical | AL | Pin | Yes | ZY | 0.3 | 1 | 75 | phenitoin | 5 | 16-20 | 199 | 300 | 0.86 | 0.37 | 1.23 | 0.16 | N/A | N/A | N/A | N/A | --- |
| Ex.4 | 1 | vertical | AL | Pin | Yes | ZY | 0.3 | 2 | 35 | phenitoin | 5 | 16-20 | 199 | 330 | 0.48 | 0.22 | 0.70 | 0.24 | N/A | N/A | N/A | N/A | --- |
| Ex.5 | 1 | vertical | AL | Pin | Yes | ZY | 0.3 | 2 | 50 | phenitoin | 5 | 16-20 | 194 | 180 | 0.48 | 0.17 | 0.65 | 0.13 | N/A | N/A | N/A | N/A | --- |
| Ex.6 | 1 | vertical | AL | Pin | Yes | ZY | 0.3 | 2 | 75 | phenitoin | 5 | 16-20 | 199 | 90 | 0.64 | 0.35 | 0.99 | 0.24 | N/A | N/A | N/A | N/A | --- |
| Ex.7 | 1 | vertical | AL | Pin | Yes | ZY | 0.3 | 2 | 90 | phenitoin | 5 | 16-20 | 198 | 90 | 1.25 | 0.16 | 1.41 | 0.67 | 0.18 | 1.07 | 0.00 | 0.01 | 3.34 |
| Ex.8 | 1 | vertical | AL | Pin | Yes | ZY | 0.3 | 6 | 75 | phenitoin | 5 | 16-20 | 185 | 60 | 1.20 | 0.34 | 1.54 | 0.57 | 0.09 | 0.00 | 0.01 | 0.03 | 2.24 |
| Ex.9 | 1 | vertical | AL | Pin | Yes | ZY | 0.5 | 2 | 90 | phenitoin | 5 | 16-20 | 199 | 180 | 1.44 | 0.34 | 1.78 | 0.46 | N/A | N/A | N/A | N/A | --- |
| Ex.10 | 1 | vertical | AL | Pin | Yes | ZY | 0.5 | 4 | 75 | phenitoin | 5 | 16-20 | 240 | 45 | 1.11 | 0.32 | 1.43 | 0.42 | 0.07 | 0.03 | 0.00 | 0.01 | 1.96 |
| Ex.11 | 1 | vertical | AL | Pin | Yes | ZY | 0.8 | 2 | 75 | phenitoin | 5 | 16-20 | 203 | 300 | 1.91 | 0.27 | 2.18 | 0.74 | 0.09 | 0.04 | 0.01 | 0.01 | 3.07 |
| Ex.12 | 1 | vertical | AL | Pin | Yes | ZY | 0.3 | 2 | 35 | phenitoin | 20 | 16-20 | 195 | 600 | 0.31 | 0.13 | 0.44 | 0.31 | N/A | N/A | N/A | N/A | --- |
| Ex.13 | 1 | vertical | AL | Pin | Yes | ZY | 0.3 | 2 | 50 | phenitoin | 30 | 16-20 | 199 | 225 | 0.52 | 0.20 | 0.72 | 0.21 | N/A | N/A | N/A | N/A | --- |
| Ex.14 | 1 | vertical | AL | Pin | Yes | ZY | 0.3 | 2 | 75 | phenitoin | 30 | 16-20 | 194 | 90 | 0.77 | 0.32 | 1.09 | 0.20 | N/A | N/A | N/A | N/A | --- |
| Ex.15 | 1 | vertical | AL | Pin | Yes | ZY | 0.3 | 2 | 50 | phenitoin | 40 | 16-20 | 200 | 330 | 0.71 | 0.23 | 0.94 | 0.25 | N/A | N/A | N/A | N/A | --- |
| Ex.16 | 1 | vertical | AL | Pin | Yes | ZY | 0.3 | 2 | 75 | phenitoin | 40 | 16-20 | 191 | 90 | 0.84 | 0.33 | 1.17 | 0.21 | N/A | N/A | N/A | N/A | --- |
| Ex.17 | 1 | vertical | AL | Pin | Yes | ZY | 0.3 | 2 | 50 | phenitoin | 40 | 16-20 | 202 | 60 | 1.06 | 0.42 | 1.48 | 0.32 | 0.12 | 0.35 | 0.02 | 0.00 | 2.29 |
| Ex.18 | 1 | vertical | AL | Pin | Yes | ZY | 0.2 | 2 | 75 | sulfametoxazole | 5 | 16-17 | 198 | 210 | 1.18 | 0.30 | 1.48 | 0.60 | 0.08 | 1.79 | 0.00 | 0.00 | 3.95 |
| Ex.19 | 1 | vertical | AL | Pin | Yes | ZY | 0.3 | 2 | 75 | sulfametoxazole | 5 | 16-17 | 193 | 270 | 0.98 | 0.32 | 1.30 | 0.24 | N/A | N/A | N/A | N/A | --- |
| Ex.20 | 1 | vertical | AL | Pin | Yes | ZY | 0.3 | 2 | 90 | sulfametoxazole | 5 | 16-17 | 193 | 240 | 1.16 | 0.44 | 1.60 | 0.29 | 0.08 | 0.06 | 0.01 | 0.01 | 2.05 |
| Ex.21 | 1 | vertical | AL | Pin | Yes | ZY | 0.3 | 2 | 50 | mefenamic acid | 5 | 12-13 | 187 | 180 | 0.65 | 0.20 | 0.85 | 0.22 | N/A | N/A | N/A | N/A | --- |
| Ex.22 | 1 | vertical | AL | Pin | Yes | ZY | 0.3 | 2 | 75 | fenofibrate | 5 | 32-33 | 196 | 210 | 0.79 | 0.30 | 1.09 | 0.26 | N/A | N/A | N/A | N/A | --- |
| Ex.23 | 1 | vertical | AL | Pin | Yes | ZY | 0.3 | 2 | 90 | fenofibrate | 5 | 32-33 | 200 | 180 | 1.17 | 0.41 | 1.58 | 0.54 | 0.07 | 0.04 | 0.01 | 0.01 | 2.25 |
| Ex.24 | 1 | vertical | AL | Pin | Yes | ZY | 0.3 | 6 | 75 | fenofibrate | 5 | 32-33 | 199 | 60 | 0.94 | 0.31 | 1.25 | 0.39 | 0.07 | 0.03 | 0.02 | 0.04 | 1.80 |
| Ex.25 | 1 | vertical | AL | Pin | Yes | ZY | 0.3 | 2 | 75 | itraconazole | 5 | 15-16 | 193 | 150 | 0.35 | 0.35 | 0.70 | 0.36 | N/A | N/A | N/A | N/A | --- |
| Ex.26 | 2 | vertical | AL | Pin | Yes | ZY | 0.3 | 4 | 50 | phenitoin | 5 | 16-20 | 260 | 30 | 0.33 | 0.15 | 0.48 | 0.10 | 0.07 | 1.15 | 0.00 | 0.01 | 1.81 |
| Ex.27 | 2 | vertical | AL | Pin | Yes | ZY | 0.3 | 4 | 50 | phenitoin | 5 | 16-20 | 197 | 90 | 0.59 | 0.21 | 0.80 | 0.21 | 0.08 | 2.43 | 0.00 | 0.03 | 3.55 |
| Ex.28 | 3 | vertical | AL | Pin | No | ZY | 0.3 | 2 | 75 | phenitoin | 40 | 16-20 | 200 | 180 | 0.71 | 0.23 | 0.94 | 0.25 | 0.15 | 0.00 | 0.00 | 0.00 | 1.34 |
| Ex.29 | 3 | vertical | AL | Pin | No | ZY | 0.3 | 4 | 75 | phenitoin | 5 | 16-20 | 180 | 90 | 0.62 | 0.23 | 0.85 | 0.19 | 0.15 | 0.01 | 0.03 | 0.00 | 1.23 |
| Ex.30 | 3 | vertical | AL | Pin | No | ZY | 0.5 | 2 | 50 | phenitoin | 5 | 16-20 | 200 | 300 | 0.85 | 0.22 | 1.07 | 0.31 | 0.08 | 0.51 | 0.02 | 0.00 | 1.99 |
| Ex.31 | 4 | Horizontal | SLS | Plate | Yes | ZY | 0.5 | 4 | 75 | phenitoin | 5 | 16-20 | 210 | 70 | 1.60 | 0.48 | 2.08 | 0.00 | 1.98 | 0.00 | 0.13 | 0.00 | 4.19 |
| Comp. Ex. 1 | 1 | vertical | AL | Pin | Yes | AL | 0.3 | 2 | 75 | phenitoin | 5 | 16-20 | 194 | 75 | 1.46 | 0.10 | 1.56 | 97.20 | N/A | N/A | N/A | N/A | --- |
| Comp. Ex.2 | 1 | vertical | AL | Pin | Yes | ZY | 1 | 2 | 75 | phenitoin | 5 | 16-20 | 201 | 420 | 8.31 | 0.69 | 9.00 | 2.34 | 0.07 | 0.06 | 0.02 | 0.03 | 11.52 |
| Comp. Ex.3 | 1 | vertical | AL | Pin | Yes | ZY | 1 | 4 | 75 | phenitoin | 5 | 16-20 | 209 | 120 | 51.12 | 3.37 | 54.49 | 10.19 | 0.23 | 0.03 | 0.05 | 0.18 | 65.17 |
| Comp. Ex.4 | 1 | vertical | AL | Pin | Yes | ZY | 0.5 | 6 | 75 | phenitoin | 5 | 16-20 | 203 | 45 | 6.33 | 0.77 | 7.10 | 1.48 | 0.09 | 0.10 | 0.00 | 0.09 | 8.86 |
| Comp. Ex.5 | 1 | vertical | AL | Pin | Yes | ZY | 0.3 | 12 | 75 | phenitoin | 5 | 16-20 | 209 | 10 | 13.63 | 1.07 | 14.70 | 2.43 | 0.07 | 0.01 | 0.00 | 0.16 | 17.37 |
| Comp. Ex.6 | 1 | vertical | AL | Pin | Yes | ZY | 0.3 | 8 | 75 | phenitoin | 5 | 16-20 | 200 | 20 | 4.73 | 0.58 | 5.31 | 1.15 | 0.06 | 0.02 | 0.01 | 0.09 | 6.64 |

Notes: ZY: partially stabilized zirconia; AL: reinforced alumina; SLS: stainless steel; N/A: not measured

Examples 1 to 25 show the results obtained by grinding 500 g of slurry until the final particle size reaches 200 nanometers in the Apparatus 1 having effective internal volume of 150 ml. The ZY concentration in the slurry was 5 ppm or less in each case (see column of "ZrY total" in the table). The processing time was in the industrially appropriate range. Further, even at the slurry concentration was as high as 50 wt%, the process was enabled without extending the processing time, and the ZY concentration was as low as 1.48 ppm (Example 17).

Examples 26 and 27 show the results obtained by grinding phenytoin in the Apparatus 2 having effective internal volume of 150 ml. In Example 26, the ZY concentration was as low as 0.48 ppm.

Examples 28 to 30 show the results obtained by grinding phenytoin in the Apparatus 3 having an internal volume of 150 ml, which has almost the same configuration in the mill as that of Apparatus 1. In all the Examples, the ZY concentration was low (1.07 ppm at the maximum), and the processing time was up to 300 minutes, which was an appropriate range. Since the Apparatus 3 has no mechanical seal, the concentrations of nickel and tungsten were lower than those in the case of using the Apparatus 1 having a mechanical seal (Examples 1 to 25). Thus, a bead mill having no mechanical seal also shows the effect of reducing the concentration of heavy metal contaminants in the slurry from the metal parts.

Example 31 shows the result obtained by the grinding process using the Apparatus 4. The Apparatus 4 is a horizontal-type bead mill wherein the stirring rotor comprises a plurality of plates having a unique shape with holes. The processing time for grinding up to 210 nanometers was 70 minutes, which was no problem. Regarding the contaminants concentration, the ZY concentration was about 2.1 ppm, which was higher than that when using the Apparatus 1 under the same operating conditions (about 1.4 ppm in Example 10), but the result was sufficiently good.

On the other hand, in Comparative Example 1 using the beads made of reinforced alumina, the contamination with aluminum in the slurry was close to 100 ppm, and thus, the concentration of the contaminants was extremely high. This is because reinforced alumina has high strength but low toughness, so that it suffered wears quickly. Comparative Examples 2 to 6 show the results obtained by the process in the Apparatus 1 using partially stabilized zirconia beads but the outer peripheral speed or the bead diameter outside the condition of the present disclosure (i.e., 0.15 mm or more and no more than a value (mm) calculated by the formula $1.07 - 0.11 \times$ [outer peripheral speed of the stirring rotor (m/sec)). In all of Comparative Examples 2 to 6, the ZY concentration exceeded 5 ppm, and the maximum value was about 54.5 ppm.

[Industrial applicability]

The present disclosure is applicable to the production of organic nanoparticles such as pharmaceuticals, health foods, and X-ray contrast agents.

[Explanation of Symbols]

1  Cylinder
2  Upper lid
3  Lower lid
4  Rotating shaft
5  Stirring rotor
6  Slurry supply port
7  Slurry outlet
8  Bead separator
9  Rotating shaft pulley
10  Belt
11  Motor pulley
12  Motor
13  Mechanical seal
14  Centrifugal bead separator
15  Slurry holder
16  Connecting pipe
17  Pumping unit
18  Slurry pipe
19  Slurry pump
20  Circulation tank
21  Stirrer
22  Slurry connecting pipe
23  Screen

**Claims**

1. A method for preparing organic nano-particles, which comprises a step in which a mixture of beads having an average particle size at least 0.15 mm and no more than 0.9 mm and a slurry containing organic particles is stirred by a stirring rotor rotating at an outer peripheral speed of 7 m/sec or less in a vessel of a wet bead mill.

2. A method for preparing organic nano-particles, which comprises a step in which a mixture of beads having an average particle size at least 0.15 mm and no more than a value (mm) calculated by the formula 1.07 - 0.11 × [outer peripheral speed of the stirring rotor (m/sec)] and a slurry containing organic particles is stirred by a stirring rotor rotating at an outer peripheral speed of 7 m/sec or less in a vessel of a wet bead mill.

3. The method according to claim 1 or 2, wherein the beads comprise partially stabilized zirconia.

4. The method according to any one of claims 1 to 3, wherein a rotating shaft for rotating the stirring rotor is inserted in the vertical direction into the vessel of the wet bead mill.

5. A method for preparing organic nano-particles, which comprises a step in which a mixture of a slurry containing organic particles and beads is stirred by a stirring rotor in a vessel of a wet bead mill, wherein the vessel of the wet bead mill is a vertical-type cylindrical vessel with an opening at the top of the vessel, and wherein a rotating shaft for rotating the stirring rotor is inserted into the cylindrical vessel from the top of the vessel through the opening, and wherein the stirring rotor is connected with the rotating shaft.

6. The method according to claim 5, wherein a slurry holder is mounted above the cylindrical vessel to connect to the vessel via a connecting pipe, and wherein a rotating shaft for rotating the stirring rotor is inserted into the vessel from the top of the slurry holder through the slurry holder and the pipe, and wherein the stirring rotor is connected with the rotating shaft, and the slurry after beads separation is discharged from the lower part of the vessel.

7. The method according to claim 5 or 6, wherein the stirring rotor rotates at an outer peripheral speed of 7 m/sec or less.

8. The method according to any one of claims 5 to 7, wherein the beads have an average particle size at least 0.15 mm and no more than 0.9 mm.

9. The method according to any one of claims 5 to 7, wherein the beads have an average particle size at least 0.15 mm and no more than a value (mm) calculated by the formula 1.07 - 0.11 × [outer peripheral speed of the stirring rotor (m/sec)].

10. The method according to any one of claims 5 to 9, wherein the beads comprise partially stabilized zirconia.

11. Organic nanoparticles obtained by the method according to any one of claims 1 to 10.

Fig. 1

Fig. 2

Fig. 3

Fig. 4

Fig. 5

Fig. 6

Fig. 7

Fig. 8

Fig. 9

Fig. 10

| | INTERNATIONAL SEARCH REPORT | International application No. |
|---|---|---|
| | | PCT/JP2020/030862 |

**A. CLASSIFICATION OF SUBJECT MATTER**
B02C 25/00(2006.01)i; C07D 233/74(2006.01)i; C07D 261/16(2006.01)i; C07D 405/14(2006.01)i; B02C 17/16(2006.01)i; B02C 17/20(2006.01)i; B02C 23/06(2006.01)i; A61K 31/196(2006.01)i; A61K 31/216(2006.01)i; A61K 31/4166(2006.01)i; A61K 31/496(2006.01)i; A61K 31/63(2006.01)i
FI:        B02C17/20; B02C17/16 B; B02C23/06; B02C25/00 C; A61K31/4166;
           A61K31/63; A61K31/216; A61K31/196; A61K31/496; C07D233/74;
           C07D261/16; C07D405/14

According to International Patent Classification (IPC) or to both national classification and IPC

**B. FIELDS SEARCHED**

Minimum documentation searched (classification system followed by classification symbols)
B02C25/00; C07D233/74; C07D261/16; C07D405/14; B02C17/16; B02C17/20; B02C23/06; A61K31/196; A61K31/216; A61K31/4166; A61K31/496; A61K31/63

Documentation searched other than minimum documentation to the extent that such documents are included in the fields searched
Published examined utility model applications of Japan          1922–1996
Published unexamined utility model applications of Japan        1971–2020
Registered utility model specifications of Japan                1996–2020
Published registered utility model applications of Japan        1994–2020

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 3222139 U (HIROSHIMA METAL & MACHINERY CO., LTD.) 11.07.2019 (2019-07-11) paragraphs [0030], [0037], fig. 1 | 5–6, 8, 11 |
| Y | paragraphs [0030], [0037], fig. 1 | 1–4, 7, 9–10 |
| Y | JP 2006-212489 A (ASHIZAWA FINETECH LTD.) 17.08.2006 (2006-08-17) paragraph [0017] | 1–4, 7, 9 |
| Y | JP 11-057514 A (NIPPON SHOKUBAI CO., LTD.) 02.03.1999 (1999-03-02) paragraph [0042] | 1–4, 7, 9 |
| Y | JP 2011-068544 A (TOSOH CORP.) 07.04.2011 (2011-04-07) paragraph [0068] | 3, 10 |

☒ Further documents are listed in the continuation of Box C.          ☒ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 07 October 2020 (07.10.2020) | 20 October 2020 (20.10.2020) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

22

**INTERNATIONAL SEARCH REPORT**

International application No.

PCT/JP2020/030862

C (Continuation).  DOCUMENTS CONSIDERED TO BE RELEVANT

| Category* | Citation of document, with indication, where appropriate, of the relevant passages | Relevant to claim No. |
|---|---|---|
| A | WO 2011/059074 A1 (MORIROKU CHEMICALS COMPANY, LTD.) 19.05.2011 (2011-05-19) | 1-11 |

Form PCT/ISA/210 (continuation of second sheet) (January 2015)

PCT/JP2020/030862

INTERNATIONAL SEARCH REPORT

Information on patent family members

International application No.

PCT/JP2020/030862

| Patent Documents referred in the Report | Publication Date | Patent Family | Publication Date |
|---|---|---|---|
| JP 3222139 U | 11 Jul. 2019 | (Family: none) | |
| JP 2006-212489 A | 17 Aug. 2006 | (Family: none) | |
| JP 11-057514 A | 02 Mar. 1999 | US 6068828 A<br>column 10, lines 46-52<br>EP 884280 A1 | |
| JP 2011-068544 A | 07 Apr. 2011 | US 2011/0244239 A1<br>paragraph [0084]<br>WO 2010/067782 A1<br>EP 2377816 A1<br>CN 102245512 A<br>KR 10-2011-0093890 A | |
| WO 2011/059074 A1 | 19 May 2011 | US 2012/0289559 A1<br>EP 2535114 A1 | |

Form PCT/ISA/210 (patent family annex) (January 2015)

**EP 4 015 086 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP H08501073 A
- JP 2016084294 A
- JP 2010510988 A
- JP 2006212489 A
- JP 2003175341 A